# FASCICULE DE BREVET EUROPEEN

(11) **EP 1 307 424 B1**
(45) Date de publication et mention de la délivrance du brevet: **04.10.2006**
(21) Numéro de dépôt: 01963066.4
(22) Date de dépôt: 07.08.2001
(51) Int. Cl.: C07C 213/08

(54) **PROCEDE DE FABRICATION DE SOLUTIONS AQUEUSES DE CHLORURES DE (METH)ACRYLOYLOXYETHYLBENZYLDIMETHYLAMMONIUM**
VERFAHREN ZUR HERSTELLUNG WÄSSRIGER LÖSUNGEN VON (METH)ACRYLOYLOXYETHYLBENZYLDIMETHYLAMMONIUM CHLORIDEN
METHOD FOR MAKING AQUEOUS SOLUTIONS OF (METH)ACRYLOYLOXYETHYLBENZYLDIMETHYLAMMONIUM CHLORIDES

(30) Priorité: 11.08.2000 FR 0010568
(43) Date de publication de la demande: 07.05.2003
(73) Titulaire: Arkema France, 92800 Puteaux (FR)
(72) Inventeur: RIONDEL, Alain, F-57600 Forbach (FR); HERBST, Gilles, F-57350 Spicheren (FR); TRETJAK, Serge, F-57520 Rouhling (FR)
(74) Mandataire: Rieux, Michel
(86) Numéro de dépôt international: PCT/FR2001/002563
(87) Numéro de publication internationale: WO 2002/014258

(56) Documents cités:
- WO-A-89/07588
- FR-A- 2 788 767
- TECHNIQUES DE L'ING¹NIEUR, G¹NIE DES PROC¹D¹S - R¹ACTEURS - PROCD¹S INDUSTRIELS, vol. J3, pages 7-12,

## Description

La présente invention porte sur un procédé de fabrication de solutions aqueuses des sels quaternaires chlorure d'acryloyloxyéthylbenzyldiméthylammonium (ADAMQUAT BZ) et chlorure de méthacryloyloxyéthylbénzyldiméthylammonium (MADAMQUAT BZ) par réaction, en présence d'eau, respectivement de l'acrylate de N,N-diméthylaminoéthyle (ADAME) et du méthacrylate de N,N-diméthylaminoéthyle (MADAME) avec le chlorure de benzyle comme agent quaternisant.

On utilise des solutions aqueuses de ce sel quaternaire (qui titrent généralement 80% en poids en matières actives) pour préparer des polymères destinés à servir de floculants cationiques dans le traitement des eaux.

Le brevet européen EP-B-250 325 décrit un procédé de préparation de solutions aqueuses de sels quaternaires incluant l'ADAMQUAT BZ et le MADAMQUAT BZ, procédé selon lequel, en présence d'au moins un inhibiteur de polymérisation :
- dans une première étape (a), on fait réagir le (M) ADAME avec 5 à 20% en poids de la quantité pondérale nécessaire à la réaction de l'agent quaternisant, ou, suivant une variante (a'), avec 5 à 20% en poids, par rapport au poids du (M) ADAME, d'une solution aqueuse de sels quaternaires, laquelle comprend de 50 à 85% en poids de sels quaternaires; et
- dans une deuxième étape (b), on ajoute en continu l'eau et l'agent quaternisant jusqu'à l'obtention de la concentration souhaitée de sels quaternaires dans l'eau.

Pendant les étapes (a) et (b), on maintient la température à une valeur comprise entre 30 et 60°C. De plus, pendant les étapes (a) et (b) et en particulier à l'approche de la fin de la réaction, on maintient dans le milieu réactionnel un courant de gaz oxygéné tel que le rapport en volume (ou débit volumétrique) de gaz total à la sortie du réacteur sur le volume (ou débit volumétrique) d'oxygène introduit à l'entrée de ce même réacteur est inférieur à 100.

Ce procédé permet dé préparer des solutions aqueuses de sels quaternaires qui ont une stabilité à température ambiante supérieure à un an. Toutefois, on constate, dans ces solutions, une teneur particulièrement élevée d'impuretés, en particulier de 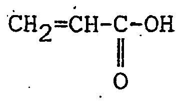 et de 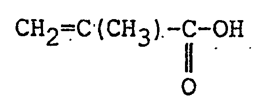 respectivement.

En outre, ce procédé nécessite des temps de réaction relativement longs, ce qui représente un inconvénient économique évident.

Dans la demande internationale WO 89/07 588, a alors été proposé un procédé destiné à réduire la formation des impuretés lors de la réaction de quaternisation. Conformément à ce procédé, la réaction est effectuée à une température comprise entre 10 et 80°C, et
- dans une première étape, on introduit dans le réacteur la totalité ou une partie de l' agent quaternisant nécessaire à la réaction, cet agent étant à l'état liquide dans les conditions de là réaction, ensuite, on ajoute du (M) ADAME, et
- dès que 0 à 30% de la stoechiométrie de l'ADAME ont été introduits dans le réacteur, on ajoute en continu et simultanément le reste d'agent quaternisant, le reste du (M) ADAME et l'eau jusqu'à l'obtention de la concentration souhaitée de sels quaternaires,
- et, dans le cas où l'agent quaternisant est introduit à l'état gazeux à la température de réaction, la réaction est effectuée en présence d'oxygène et on impose une pression de manière que l'agent quaternisant soit liquide à la température de réaction, et, en fin de réaction, on diminue progressivement la pression jusqu'à la pression atmosphérique et simultanément on impose un rapport en débit volumétrique de gaz total à la sortie du réacteur sur le débit volumétrique d'oxygène introduit dans le réacteur inférieur à 100.

Le procédé ci-dessus selon WO 89/07 588 apporte des améliorations notables au procédé selon EP-B-250 325. Cependant, il est apparu que la pureté avec laquelle on obtient les sels quaternaires est encore insuffisante. Ainsi, ce procédé permet de réduire significativement la teneur en acide (méth)acrylique issue de l'hydrolyse du (M) ADAME. En revanche, il conduit à un (M) ADAMQUAT BZ contenant une quantité importante d'alcool benzylique (500 à 2600 ppm), impureté résultant de l'hydrolyse du chlorure de benzyle et qui rend le (M)ADAMQUAT BZ impropre à son utilisation en polymérisation.

Par ailleurs, le document FR 2 788 767 décrit un procédé de préparation de solutions aqueuses de sels insaturés d'ammonium quaternaire, incluant l'ADAMQUAT BZ, procédé selon lequel la réaction est conduite dans un réacteur tubulaire avec introduction de l'agent quaternisant en pied de réacteur et introduction de l'ADAME et de l'eau en tête de réacteur. La réaction a lieu sous une pression allant de 10 à 20 bars.

La Société déposante a donc recherché des conditions opératoires de préparation de solutions aqueuses d'ADAMQUAT BZ et de MADAMQUAT BZ, qui soient capables de minimiser des impuretés précitées, de façon à proposer ce sel en solution aqueuse de très haute qualité analytique, tout en réduisant la durée de la réaction.

Ce nouveau procédé, qui fait donc l'objet de la présente invention, est caractérisé par le fait que l'on conduit la réaction en continu à la pression atmosphérique dans un réacteur fonctionnant selon la technologie du réacteur parfaitement agité (cf. Techniques de l'Ingénieur, Volume J3 Génie des procédés Réacteurs - Procédés Industriels).

Par rapport aux procédés connus en batch, le procédé de l'invention permet d'améliorer la productivité, de réduire le volume réactionnel et d'améliorer ainsi la sécurité, tout en offrant l'avantage de fournir un produit de qualité constante.

Par ailleurs, on conduit généralement la réaction selon l'invention, avec un rapport molaire du (M)ADAME au chlorure de benzyle qui est compris entre 1 et 1,1, en particulier entre 1,02 et 1,05. Quant au rapport des débits d'introduction chlorure de benzyle / eau, il est généralement compris entre 1,8 et 2,5, en particulier entre 2 et 2,2.

La réaction selon l'invention est généralement conduite avec un temps de séjour de 1 à 8 heures, en particulier entre 2 et 6 heures. La température est généralement comprise entre 25 et 55°C, en particulier entre 35 et 50°C.

Conformément à un mode de réalisation particulièrement préféré, on remplit initialement le réacteur avec une solution aqueuse de chlorure de (méth) acryloyloxyéthylbenzyldiméthylammonium à 75-80% en poids et on introduit ensuite simultanément l'eau, le (M)ADAME et le chlorure de benzyle.

Le procédé selon la présente invention est par ailleurs avantageusement conduit en présence d'au moins un stabilisant, choisi notamment parmi le 3,5-ditert.-butyl-4-hydroxytoluène, l'éther méthylique d'hydroquinone, l'hydroquinone, le catéchol, le tert.-butyl catéchol et les mélanges de ces stabilisants, la teneur en agent(s) stabilisante(s) étant notamment de 20 à 2000 ppm, de préférence de 100 à 1200 ppm par rapport à la solution aqueuse de chlorure dé (méth)acryloyloxyéthylbenzyldiméthylammonium.

Le procédé selon l'invention est en outre avantageusement conduit en présence d'au moins un agent séquestrant pour métaux, choisi notamment parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique, la teneur en agent(s) séquestrant(s) étant notamment de 1 à 100 ppm, de préférence de 5 à 30 ppm, par rapport à la solution aqueuse de chlorure de (méth)acryloyloxyéthylbenzyldiméthylammonium.

D'une manière générale, les agents séquestrants sont ajoutés sous la forme d'une solution aqueuse, car ils sont généralement disponibles sous cette forme. Ainsi, le sel pentasodique de l'acide diéthylène triamine penta acétique commercialisé sous la dénomination VERSENEX 80 se présente sous la forme d'une solution aqueuse à 40% en poids environ.

Le procédé selon l'invention permet notamment de préparer des solutions aqueuses ayant une concentration en sels quaternaires (I) de 50 à 85% en poids, et contenant des quantités très faibles d'impuretés, comme illustré dans le Tableau ci-après.

L'Exemple suivant illustre la présente invention sans toutefois en limiter la portée.

### EXEMPLE : PRÉPARATION D'ADAMOUAT BZ

Dans un réacteur d'une capacité d'un litre, fonctionnant selon la technologie du réacteur parfaitement agité, on charge 700 g d'ADAMQUAT BZ 80 qui est porté sous bullage d'air et sous agitation à 38°C. On introduit ensuite simultanément l'ADAME (stabilisé à 800 ppm d'EMHQ), l'eau et le chlorure de benzyle à des débits respectifs visés de respectivement 75, 7 g/h (0, 529 mole/h), 31, 5 g/h et 65, 65 g/h (0, 518 mole/h), soit un rapport molaire ADAME/chlorure de benzyle de 1,02 et un rapport pondéral chlorure de benzyle / eau de 2,084.

Le débit de soutirage du produit fini par débordement est de 171,7 g/h, soit un temps de séjour global de 4 heures.

Après une mise en régime fixée arbitrairement à 6 h, soit 1,5 temps de séjour, le produit est analysé en HPLC. Il présente les caractéristiques suivantes :

| Analyse HPLC | Teneur (ppm) |
|---|---|
| Acide acrylique | 3418 |
| Chlorure de benzyle | < 10 |
| Alcool benzylique | 72 |

## Revendications

1. Procédé de fabrication d'une solution aqueuse de chlorure de (méth) acryloyloxyéthylbenzyldiméthylammonium ((M)ADAMQUAT BZ), par réaction, en présence d'eau, du (méth) acrylate de N, N-diméthylaminoéthyle ((M) ADAME) avec le chlorure de benzyle, **caractérisé par le fait que** l'on conduit la réaction en continu à la pression atmosphérique dans un réacteur fonctionnant selon la technologie du réacteur parfaitement agité.

2. Procédé selon la revendication 1, **caractérisé par le fait que** l'on conduit la réaction avec un rapport molaire du (M)ADAME au chlorure de benzyle qui est compris entre 1 et 1,1, en particulier entre 1,02 et 1,05.

3. Procédé selon l'une des revendications 1 et 2, **caractérisé par le fait que** l'on conduit la réaction avec un rapport des débits d'introduction chlorure de benzyle / eau compris entre 1,8 et 2,5, en particulier entre 2 et 2,2.

4. Procédé selon l'une des revendications 1 à 3, **caractérisé par le fait que** l'on conduit la réaction avec un temps de séjour de 1 à 8 heures, en particulier entre 2 et 6 heures.

5. Procédé selon l'une des revendications 1 à 4, **caractérisé par** le faut que l'on conduit la réaction à une température de 25 à 55°C, en particulier de 35 à 50°C.

6. Procédé selon l'une des revendications 1 à 5, **caractérisé par le fait qu'**on remplit initialement le réacteur avec une solution aqueuse de chlorure de (méth)acryloyloxyéthylbenzyldiméthylammonium à 75-80% en poids et on introduit ensuite simultanément l'eau, le (M)ADAME et le chlorure de benzyle.

7. Procédé selon l'une des revendications 1 à 6, **caractérisé par le fait qu'**il est conduit en présence d'au moins un stabilisant, choisi notamment parmi le 3, 5-ditert. butyl-4-hydroxytoluène, l'éther méthylique d'hydroquinone, l'hydroquinone, le catéchol, le tert.-butyl catéchol et les mélanges de ces stabilisants, la teneur en agent(s) stabilisant (s) étant notamment de 20, à 200 ppm, de préférence de 100 à 1200 ppm par rapport à la solution aqueuse de chlorure de (méth)acryloyloxyéthylbenzyldiméthylammonium.

8. Procédé selon l'une des revendications 1 à 7, **caractérisé par le fait qu'**il est conduit en outre en présence d'au moins un agent séquestrant pour métaux, choisi notamment parmi l'acide diéthylène triamine penta acétique, le sel pentasodique de l'acide diéthylène triamine penta acétique, l'acide N-hydroxyéthyl-éthylène diamine triacétique, et le sel trisodique de l'acide N-hydroxyéthyl-éthylène diamine triacétique, la teneur en agent(s) séquestrant (s) étant notamment de 1 à 100 ppm, de préférence de 5 à 30 ppm, par rapport à la solution aqueuse de chlorure de (méth)acryloyloxyéthylbenzyldiméthylammonium.

## Claims

1. Process for the manufacture of an aqueous (meth)-acryloyloxyethylbenzyldimethylammonium chloride ((M)ADAMQUAT BZ) solution by reaction, in the presence of water, of N,N-dimethylaminoethyl (meth)acrylate ((M)ADAME) with benzyl chloride, **characterized in that** the reaction is carried out continuously at atmospheric pressure in a reactor operating according to the perfectly stirred reactor technology.

2. Process according to Claim 1, **characterized in that** the reaction is carried out with a molar ratio of the (M)ADAME to the benzyl chloride which is between 1 and 1.1, in particular between 1.02 and 1.05.

3. Process according to either of Claims 1 and 2, **characterized in that** the reaction is carried out with a ratio of the benzyl chloride/water introduction flow rates of between 1.8 and 2.5, in particular between 2 and 2.2.

4. Process according to one of Claims 1 to 3, **characterized in that** the reaction is carried out with a residence time of 1 to 8 hours, in particular between 2 and 6 hours.

5. Process according to one of Claims 1 to 4, **characterized in that** the reaction is carried out at a temperature of 25 to 55°C, in particular of 35 to 50°C.

6. Process according to one of Claims 1 to 5, **characterized in that** the reactor is initially filled with a 75-80% by weight aqueous (meth)acryloyloxyethylbenzyldimethylammonium chloride solution and, subsequently, water, (M)ADAME and benzyl chloride are introduced simultaneously.

7. Process according to one of Claims 1 to 6, **characterized in that** it is carried out in the presence of at least one stabilizing agent chosen in particular from 3,5-di(tert-butyl)-4-hydroxytoluene, hydroquinone methyl ether, hydroquinone, catechol, tert-butylcatechol and mixtures of these stabilizing agents, the content of stabilizing agent(s) being in particular from 20 to 2000 ppm, preferably from 100 to 1200 ppm, with respect to the (meth)acryloyloxyethylbenzyldimethylammonium chloride solution.

8. Process according to one of Claims 1 to 7, **characterized in that** it is carried out in addition in the presence of at least one metal-sequestering agent chosen in particular from diethylenetriaminepentaacetic acid, the pentasodium salt of diethylenetriaminepentaacetic acid, N-(hydroxyethyl)ethylenediaminetriacetic acid and the trisodium salt of N-(hydroxyethyl)ethylenediaminetriacetic acid, the content of sequestering agent(s) being in particular from 1 to 100 ppm, preferably from 5 to 30 ppm, with respect to the aqueous (meth)acryloyloxyethylbenzyldimethylammonium chloride solution.

## Patentansprüche

1. Verfahren zur Herstellung einer wäßrigen Lösung von (Meth)acryloyloxyethylbenzyldimethylammoniumchlorid ((M)ADAMQUAT BZ) durch Umsetzung von N,N-Dimethylaminoethyl(meth)acrylat ((M)ADAME) mit Benzylchlorid in Gegenwart von Wasser, **dadurch gekennzeichnet, daß** man die Umsetzung bei Normaldruck in einem nach der Technologie des perfekten Rührreaktors arbeitenden Reaktor durchführt.

2. Verfahren nach Anspruch 1, **dadurch gekennzeichnet, daß** man die Umsetzung mit einem Molverhältnis von (M)ADAME zu Benzylchlorid zwischen 1 und 1,1 und insbesondere zwischen 1,02 und 1,05 durchführt.

3. Verfahren nach Anspruch 1 oder 2, **dadurch gekennzeichnet, daß** man die Umsetzung mit einem Benzylchlorid/Wasser-Eintragsstromverhältnis zwischen 1,8 und 2,5 und insbesondere zwischen 2 und 2,2 durchführt.

4. Verfahren nach einem der Ansprüche 1 bis 3, **dadurch gekennzeichnet, daß** man die Umsetzung mit einer Verweilzeit von 1 bis 8 Stunden und insbesondere zwischen 2 und 6 Stunden durchführt.

5. Verfahren nach einem der Ansprüche 1 bis 4, **dadurch gekennzeichnet, daß** man die Umsetzung bei einer Temperatur von 25 bis 55°C und insbesondere 35 bis 50°C durchführt.

6. Verfahren nach einem der Ansprüche 1 bis 5, **dadurch gekennzeichnet, daß** man zunächst im Reaktor eine 75-80 gew.-%ige wäßrige Lösung von (Meth)acryloyloxyethylbenzyldimethylammoniumchlorid vorlegt und dann gleichzeitig das Wasser, das (M)ADAME und das Benzylchlorid einträgt.

7. Verfahren nach einem der Ansprüche 1 bis 6, **dadurch gekennzeichnet, daß** man es in Gegenwart mindestens eines Stabilisators, der insbesondere unter 3,5-Di-tert.-butyl-4-hydroxytoluol, Hydrochinonmethylether, Hydrochinon, Brenzcatechin, tert.-Butylbrenzcatechin und Gemischen dieser Stabilisatoren ausgewählt ist, durchführt, wobei der Stabilisatorgehalt insbesondere 20 bis 2000 ppm und vorzugsweise 100 bis 1200 ppm, bezogen auf die wäßrige Lösung von (Meth)acryloyloxyethylbenzyldimethylammoniumchlorid, beträgt.

8. Verfahren nach einem der Ansprüche 1 bis 7, **dadurch gekennzeichnet, daß** man es außerdem in Gegenwart mindestens eines Metallsequestriermittels, das insbesondere unter Diethylentriaminpentaessigsäure, dem Pentanatriumsalz von Diethylentriaminpentaessigsäure, N-(Hydroxyethyl)-ethylendiamintriessigsäure und dem Trinatriumsalz von N-(Hydroxyethyl)ethylendiamintriessigsäure ausgewählt ist, wobei der Sequestriermittelgehalt insbesondere 1 bis 100 ppm und vorzugsweise 5 bis 30 ppm, bezogen auf die wäßrige Lösung von (Meth)-acryloyloxyethylbenzyldimethylammoniumchlorid, beträgt.
